# EUROPEAN PATENT SPECIFICATION

(11) **EP 3 461 393 B1**
(45) Date of publication and mention of the grant of the patent: **17.06.2020**
(21) Application number: 18197594.7
(22) Date of filing: 28.09.2018
(51) Int. Cl.: A61B 1/227, A61B 1/00

(54) **METHOD FOR GUIDING A USER TO OBTAIN AN EARDRUM STANDARD IMAGE**
VERFAHREN ZUR FÜHRUNG EINES BENUTZERS ZUR ERFASSUNG EINES TROMMELFELLSTANDARDBILDES
PROCÉDÉ POUR GUIDER UN UTILISATEUR AFIN D'OBTENIR UNE IMAGE STANDARD DANS LA MEMBRANE DU TYMPAN

(30) Priority: 30.09.2017 TW 106133945
(43) Date of publication of application: 03.04.2019
(73) Proprietor: SyncVision Technology Corp., 24158 New Taipei City (TW)
(72) Inventor: Wang, Pa-Chune, Taipei City (TW); Fang, Te-Yung, Taipei City (TW)
(74) Representative: Straus, Alexander

(56) References cited:
- US-A1- 2002 038 076
- US-A1- 2015 065 803
- US-A1- 2015 351 606

## Description

### FIELD OF THE INVENTION

The present invention relates to a method for guiding a user to obtain an eardrum standard image. The present invention also relates to an otoscope suitable for implementing said method.

### BACKGROUND OF THE INVENTION

A group of middle ear inflammatory diseases are collectively referred to as otitis media, the two most common types of which are acute otitis media (AOM) and acute otitis media with effusion (OME). Patients with AOM may have fever and feel pain, and usually accompanied with hearing impairment. If not completely cured, AOM may result in otitis media with effusion or chronic suppurative otitis media. Some studies showed that if young children suffer a long term otitis media, the hearing impairment may affect their ability to learn. US Patent US2015065803 discloses apparatuses and methods for mobile imaging and image analysis for assisting in the acquisition and analysis of images of the tympanic membrane. These apparatuses may guide or direct a subject in taking an image of a tympanic membrane, including automatically detecting which direction to adjust the position of the apparatus to capture an image and automatically indicate when the tympanic membrane has been imaged. US Patent US2002038076 discloses a portable data collection device provided for diagnostic image and data collection at a remote location. The device is implemented as an otoscope including a speculum and light source for illumination of the ear canal. A digital camera element collects the reflected images and provides the images to a processor. US Patent US2015351606 discloses a method of identifying objects in a subject's ear, comprising the following steps: introducing an optical electronic imaging unit and a light source into an ear canal of a subject's outer ear, wherein an optical axis especially directs at the eardrum of the subject's ear; using the electronic imaging unit to inter alia identify the objects, especially the eardrum.

Typically, the diagnosis of otitis media requires visual inspection of the eardrum by professional medical personnel. There is still a need for a method or device for assisting professional medical personnel or an ordinary person to obtain an eardrum standard image for the diagnosis of otitis media.

### SUMMARY OF THE INVENTION

In one aspect, the present invention provides a method for guiding a user to obtain an eardrum standard image, comprising providing an otoscope including an imaging module, a computing module, and a viewing module; showing on the viewing module a circle sight to assist the user in capturing an eardrum image using the imaging module; the computing module determining an eardrum region in the eardrum image, and calculating a percentage of area of overlap of the circle sight with the eardrum region; and if the percentage is higher than a first pre-determined value, showing a first sign on the viewing module to inform the user. The method further comprises the step: if the percentage is lower than a second pre-determined value, showing a second sign on the viewing module to guide the user to move the otoscope toward a specific direction.

In certain embodiments of the present invention, the circle sight shown on the viewing module comprises an inner sight for aiming at auditory ossicles.

In a further embodiment of the present invention, the eardrum region may be determined by a process comprising the following steps: (i) calculating contrast values of the eardrum image, and (ii) defining a boundary between an eardrum and an ear canal in the eardrum image.

In certain embodiments of the present invention, the first pre-determined value is 20%, 25%, or 30%.

According to one embodiment of the present invention, the first sign includes a color change of the circle sight.

In certain embodiments, the second pre-determined value is at least 70%, 80%, 90%, or 95%. In addition, the second sign may include an arrow indicating the specific direction.

In another aspect, the present invention provides an otoscope comprising an imaging module; a computing module; a viewing module; and a memory including instructions, which when executed by the computing module, cause the computing module to: cause the viewing module to show a circle sight to assist a user in capturing an eardrum image using the imaging module; determine an eardrum region in the eardrum image, and calculate a percentage of area of overlap of the circle sight with the eardrum region; and if the percentage is higher than a first pre-determined value, cause the viewing module to show a first sign to inform the user. The instructions, when executed by the computing module, further cause the computing module to: if the percentage is lower than a second pre-determined value, cause the viewing module to show a second sign to guide the user to move the otoscope toward a specific direction.

In certain embodiments of the present invention, the circle sight shown on the viewing module comprises an inner sight for aiming at auditory ossicles.

According to a further embodiment of the present invention, the eardrum region may be determined by a process comprising the steps of calculating contrast values of the eardrum image, and defining a boundary between an eardrum and an ear canal in the eardrum image. In certain embodiments of the present invention, the first pre-determined value is 20%, 25%, or 30%.

According to one embodiment of the present invention, the first sign includes a color change of the circle sight.

In certain embodiments, the second pre-determined value is at least 70%, 80%, 90%, or 95%.

In addition, the second sign may include an arrow indicating the specific direction.

These and other aspects will become apparent from the following description of the preferred embodiment taken in conjunction with the following drawings, although variations and modifications therein may be affected without departing from the spirit and scope of the novel concepts of the disclosure.

### BRIEF DESCRIPTION OF THE DRAWINGS

The foregoing summary, as well as the following detailed description of the invention, will be better understood when read in conjunction with the appended drawing. In the drawings:
FIG. 1 is a flow chart of a method for guiding a user to obtain an eardrum standard image according to the present invention.
FIG. 2 illustrates the calculation of a percentage of area of overlap of a circle sight with an eardrum region.
FIG. 3 shows an example of a circle sight with an inner sight.

### DESCRIPTION OF THE INVENTION

Unless defined otherwise, all technical and scientific terms used herein have the same meaning as commonly understood by a person skilled in the art to which this invention belongs.

As used herein, the singular forms "a", "an", and "the" include plural referents unless the context clearly dictates otherwise. Thus, for example, reference to "a sample" includes a plurality of such samples and equivalents thereof known to those skilled in the art.

In one aspect, the present invention provides a method for guiding a user to obtain an eardrum standard image. The method comprises the following steps: (i) providing an otoscope including an imaging module, a computing module, and a viewing module, (ii) showing on the viewing module a circle sight to assist the user in capturing an eardrum image using the imaging module, (iii) the computing module determining an eardrum region in the eardrum image, and calculating a percentage of area of overlap of the circle sight with the eardrum region, and (iv) if the percentage is higher than a first pre-determined value, showing a first sign on the viewing module to inform the user. The method further comprises the following step: if the percentage is lower than a second pre-determined value, showing a second sign on the viewing module to guide the user to move the otoscope toward a specific direction.

The computing module may be implemented in hardware and/or in software. The computing module may be an application specific integrated circuit (ASIC) and/or may be represented as machine-readable software code. The computing module may comprise one or more input terminals and one or more output terminals.

The viewing module includes but is not limited to a LCD display. The imaging module may comprise a camera.

Given that the viewing module has a resolution of 1,280 x 720, the circle sight may be centered at the position of (640, 360) and may have a radius of 40% of the height of the image (720 pixel).

In certain embodiments of the present invention, the circle sight shown on the viewing module includes an inner sight, which is adapted for aiming at auditory ossicles. For example, the inner sight may have a generally circular shape or a generally triangular shape.

According to one embodiment of the present invention, the eardrum region may be determined by a process comprising the following steps: (i) calculating contrast values of the eardrum image, and (ii) defining a boundary between an eardrum and an ear canal in the eardrum image. For example, the eardrum region may be determined through a contrast enhancement of the eardrum image by a binary threshold method to convert the eardrum image to a binary (black and white) image.

In certain embodiments of the present invention, the first pre-determined value is 20%, 25%, or 30%.

According to one embodiment of the present invention, the first sign includes a color change of the circle sight.

In certain embodiments, the second pre-determined value is at least 70%, 80%, 90%, or 95%. In addition, the second sign may include an arrow indicating the specific direction.

In another aspect, the present invention provides an otoscope suitable for implementing the above described method. The otoscope comprises an imaging module, a computing module, a viewing module, and a memory including instructions. When the instructions are executed by the computing module, they cause the computing module to: (i) cause the viewing module to show a circle sight to assist a user in capturing an eardrum image using the imaging module, (ii) determine an eardrum region in the eardrum image, and calculate a percentage of area of overlap of the circle sight with the eardrum region, and (iii) if the percentage is higher than a first pre-determined value, cause the viewing module to show a first sign to inform the user. The instructions, when executed by the computing module, further cause the computing module to: if the percentage is lower than a second pre-determined value, cause the viewing module to show a second sign to guide the user to move the otoscope toward a specific direction.

The computing module may be implemented in hardware and/or in software. The computing module may be an application specific integrated circuit (ASIC) and/or may be represented as machine-readable software code. The computing module may comprise one or more input terminals and one or more output terminals.

The viewing module includes but is not limited to a LCD display. The imaging module may comprise a camera.

Given that the viewing module has a resolution of 1,280 x 720, the circle sight may be centered at the position of (640, 360) and may have a radius of 40% of the height of the image (720 pixel).

In certain embodiments of the present invention, the circle sight shown on the viewing module comprises an inner sight for aiming at auditory ossicles. For example, the inner sight may have a generally circular shape or a generally triangular shape.

According to the present invention, the eardrum region may be determined by a process comprising the following steps: (i) calculating contrast values of the eardrum image, and (ii) defining a boundary between an eardrum and an ear canal in the eardrum image. For example, the eardrum region may be determined through a contrast enhancement of the eardrum image by a binary threshold method to convert the eardrum image to a binary (black and white) image.

In certain embodiments of the present invention, the first pre-determined value is 20%, 25%, or 30%. According to one embodiment of the present invention, the first sign includes a color change of the circle sight.

According to certain embodiments of the present invention,

In certain embodiments, the second pre-determined value is at least 70%, 80%, 90%, or 95%. In addition, the second sign may include an arrow indicating the specific direction.

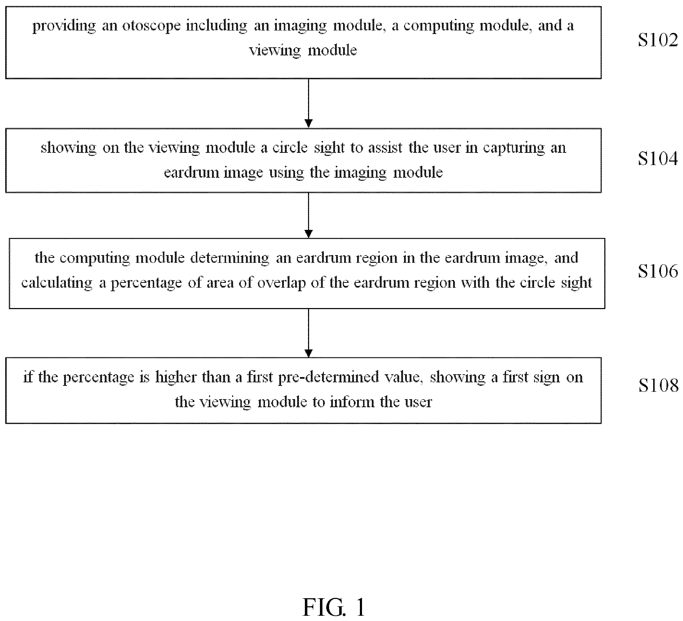

Please refer to FIG. 1. The method for guiding a user to obtain an eardrum standard image comprises the following steps. First, as shown in step S102, an otoscope is provided. The otoscope includes an imaging module, a computing module, and a viewing module. Then, a circle sight is shown on the viewing module to assist the user in capturing an eardrum image using the imaging module (step S104). As shown in step S106, the computing module then determines an eardrum region in the eardrum image, and calculates a percentage of area of overlap of the circle sight with the eardrum region. Lastly, if the percentage is higher than a first pre-determined value, a first sign is shown on the viewing module to inform the user (step S108).

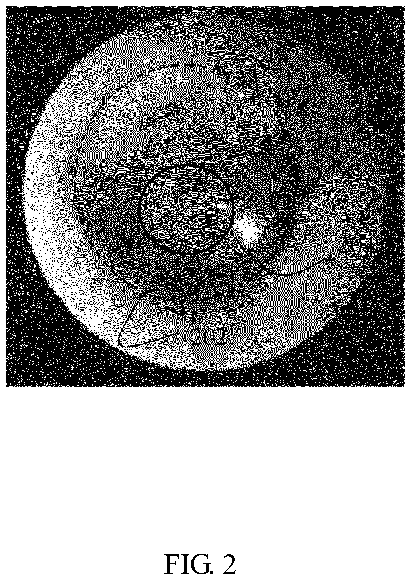

Referring FIG. 2, for an otoscope including an imaging module, a computing module and a viewing module, a circle sight 202 may be shown on the viewing module to assist a user in capturing an eardrum image. Then, the computing module determines an eardrum region 204 in the eardrum image, based on contrast values of the eardrum image. In addition, the computing module calculates a percentage of area of overlap of the circle sight 202 with the eardrum region 204. In the present example, the percentage is about twenty percent. If the percentage is higher than a first pre-determined value, a first sign may be shown on the viewing module to inform the user.

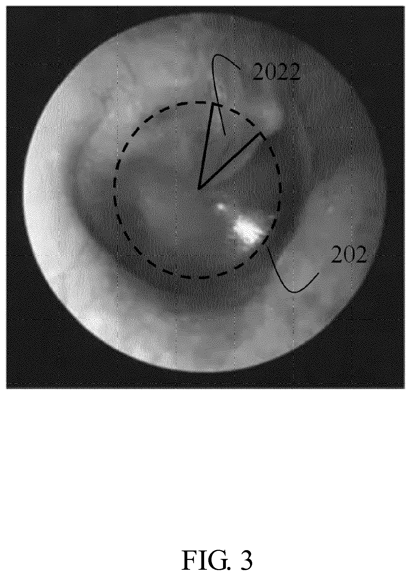

An example of a circle sight with an inner sight is shown in Fig. 3. A circle sight 202 comprises an inner sight 2022 for aiming at auditory ossicles. The inner sight 2022 has a generally triangular shape.

The purpose of capturing accurate and consistent eardrum images may be achieved by the method of the present invention. In addition to the simple operation of the otoscope, medical personnel can also establish a database with standardized eardrum images. An eardrum standard image of a patient may be compared with a plurality of eardrum standard images in the database associated with otitis media to assist diagnosis.

In one example of the present invention, the otoscope may further comprise a step motor and an otitis media database; wherein the step motor, which is connected to the detecting part and display part, is moving to the predetermined position by the difference value of the repeat area when the error display occurs. The otitis media database, which is connected to a camera module, is saving a plurality of the ear drum reference images, and is to assess whether the ear drum standard image has any corresponding feature of otitis media.

## Claims

1. A method for guiding a user to obtain an eardrum standard image, comprising:
providing an otoscope including an imaging module, a computing module, and a viewing module;
showing on the viewing module a circle sight to assist the user in capturing an eardrum image using the imaging module;
the computing module determining an eardrum region in the eardrum image, and calculating a percentage of area of overlap of the circle sight with the eardrum region;
if the percentage is higher than a first pre-determined value, showing a first sign on the viewing module to inform the user; and
if the percentage is lower than a second pre-determined value, showing a second sign on the viewing module to guide the user to move the otoscope toward a specific direction.

2. The method of claim 1, wherein the circle sight shown on the viewing module comprises an inner sight for aiming at auditory ossicles.

3. The method of claim 1, wherein the eardrum region is determined by a process comprising the steps of calculating contrast values of the eardrum image, and defining a boundary between an eardrum and an ear canal in the eardrum image.

4. The method of claim 1, wherein the first pre-determined value is 20%, 25%, or 30%.

5. The method of claim 1, wherein the first sign includes a color change of the circle sight.

6. The method of claim 1, wherein the second pre-determined value is at least 70%, 80%, 90%, or 95%.

7. The method of claim 1, wherein the second sign includes an arrow indicating the specific direction.

8. An otoscope comprising:
an imaging module;
a computing module;
a viewing module; and
a memory including instructions, which when executed by the computing module, cause the computing module to:
cause the viewing module to show a circle sight to assist a user in capturing an eardrum image using the imaging module;
determine an eardrum region in the eardrum image, and calculate a percentage of area of overlap of the circle sight with the eardrum region;
if the percentage is higher than a first pre-determined value, cause the viewing module to show a first sign to inform the user; and,
: if the percentage is lower than a second pre-determined value, cause the viewing module to show a second sign to guide the user to move the otoscope toward a specific direction.

9. The otoscope of claim 8, wherein the circle sight shown on the viewing module comprises an inner sight for aiming at auditory ossicles.

10. The otoscope of claim 8, wherein the eardrum region is determined by a process comprising the steps of calculating contrast values of the eardrum image, and defining a boundary between an eardrum and an ear canal in the eardrum image.

11. The otoscope of claim 8, wherein the first pre-determined value is 20%, 25%, or 30%.

12. The otoscope of claim 8, wherein the first sign includes a color change of the circle sight.

13. The otoscope of claim 8, wherein the second pre-determined value is at least 70%, 80%, 90%, or 95%, and the second sign includes an arrow indicating the specific direction.

## Patentansprüche

1. Verfahren zum Führen eines Nutzers beim Erhalten eines Trommelfell-Standardbildes, wobei das Verfahren umfasst:
Bereitstellen eines Otoskopes, das ein Bilderzeugungsmodul, ein Rechnermodul, und ein Anzeigemodul umfasst;
Anzeigen einer Kreisansicht auf dem Anzeigemodul, um dem Nutzer bei der Aufnahme eines Trommelfell-Bildes unter Verwendung des Bilderzeugungsmoduls zu unterstützen;
Bestimmen eines Trommelfell-Bereiches in dem Trommelfell-Bild durch das Rechnermodul, und Berechnen eines Flächenüberlappungsanteils der Kreisansicht mit dem Trommelfell-Bereich;
wobei wenn der Anteil höher ist als ein erster bestimmter Wert, ein erstes Zeichen auf dem Anzeigemodul angezeigt wird, um den Nutzer zu informieren; und
wenn der Anteil niedriger ist, als ein zweiter bestimmter Wert, ein zweites Zeichen auf dem Anzeigemodul angezeigt wird, um den Nutzer zu führen, das Otoskop in eine spezifische Richtung zu bewegen.

2. Verfahren nach Anspruch 1, worin die auf dem Anzeigemodul angezeigte Kreisansicht eine innere Ansicht zum Ausrichten an den Gehörknöchelchen umfasst.

3. Verfahren nach Anspruch 1, wobei der Trommelfell-Bereich durch einen Vorgang bestimmt wird, der die Schritte umfasst, Berechnen von Kontrastwerten des Trommelfell-Bildes, und Definieren einer Grenze zwischen einem Trommelfell und einem Ohrkanal in dem Trommelfell-Bild.

4. Verfahren nach Anspruch 1, worin der erste bestimmte Wert 20%, 25%, oder 30% ist.

5. Verfahren nach Anspruch 1, worin das erste Zeichen einen Farbwechsel der Kreisansicht umfasst.

6. Verfahren nach Anspruch 1, worin der zweite bestimmte Wert mindestens 70%, 80%, 90%, oder 95% ist.

7. Verfahren nach Anspruch 1, worin das zweite Zeichen einen Pfeil umfasst, der die spezifische Richtung anzeigt.

8. Otoskop, welches umfasst:
ein Bilderzeugungsmodul;
ein Rechnermodul;
ein Anzeigemodul; und
einen Speicher, der Befehle umfasst, wodurch, wenn diese durch das Rechnermodul ausgeführt werden, das Rechnermodul bewirkt, dass:
das Anzeigemodul eine Kreisansicht zum Unterstützen eines Nutzers bei der Aufnahme eines Trommelfell-Bildes unter Verwendung des Bilderzeugungsmoduls anzeigt;
ein Trommelfell-Bereich in dem Trommelfell-Bild bestimmt wird, und ein Flächenüberlappungsanteil der Kreisansicht mit dem Trommelfell-Bereich berechnet wird;
wobei wenn der Anteil höher ist als ein erster bestimmter Wert, das Anzeigemodul ein erstes Zeichen anzeigt, um den Nutzer zu informieren; und
wenn der Anteil niedriger ist, als ein zweiter bestimmter Wert, das Anzeigemodul ein zweites Zeichen anzeigt, um den Nutzer zu führen, das Otoskop in eine spezifische Richtung zu bewegen.

9. Otoskop nach Anspruch 8, worin die auf dem Anzeigemodul angezeigte Kreisansicht eine innere Ansicht zum Ausrichten an den Gehörknöchelchen umfasst.

10. Otoskop nach Anspruch 8, wobei der Trommelfell-Bereich durch einen Vorgang bestimmt wird, der die Schritte umfasst, Berechnen von Kontrastwerten des Trommelfell-Bildes, und Definieren einer Grenze zwischen einem Trommelfell und einem Ohrkanal in dem Trommelfell-Bild.

11. Otoskop nach Anspruch 8, worin der erste bestimmte Wert 20%, 25%, oder 30% ist.

12. Otoskop nach Anspruch 8, worin das erste Zeichen einen Farbwechsel der Kreisansicht umfasst.

13. Otoskop nach Anspruch 8, worin der zweite bestimmte Wert mindestens 70%, 80%, 90%, oder 95% ist, und das zweite Zeichen einen Pfeil umfasst, der die spezifische Richtung anzeigt.

## Revendications

1. Procédé de guidage d'un utilisateur pour obtenir une image standard de tympan, comprenant :
l'utilisation d'un otoscope comprenant un module d'imagerie, un module de calcul et un module de visualisation ;
l'affichage, sur le module de visualisation, d'un viseur circulaire pour aider l'utilisateur à capturer une image de tympan à l'aide du module d'imagerie ;
la détermination, par le module de calcul, d'une région de tympan dans l'image de tympan et le calcul d'un pourcentage de zone de chevauchement du viseur circulaire avec la région de tympan ;
si le pourcentage est supérieur à une première valeur prédéterminée, l'affichage d'un premier signe sur le module de visualisation pour informer l'utilisateur ; et
si le pourcentage est inférieur à une deuxième valeur prédéterminée, l'affichage d'un deuxième signe sur le module de visualisation pour guider l'utilisateur pour déplacer l'otoscope dans un sens spécifique.

2. Procédé selon la revendication 1, le viseur circulaire affiché sur le module de visualisation comprenant un viseur interne pour viser les osselets auditifs.

3. Procédé selon la revendication 1, la région du tympan étant déterminée par un procédé comprenant les étapes de calcul de valeurs de contraste de l'image du tympan et de définition d'une limite entre un tympan et un conduit auditif dans l'image du tympan.

4. Procédé selon la revendication 1, la première valeur prédéterminée étant de 20%, de 25% ou de 30%.

5. Procédé selon la revendication 1, le premier signe comprenant un changement de couleur du viseur circulaire.

6. Procédé selon la revendication 1, la deuxième valeur prédéterminée étant d'au moins 70%, 80%, 90% ou 95%.

7. Procédé selon la revendication 1, le deuxième signe comprenant une flèche indiquant le sens spécifique.

8. Otoscope comprenant :
un module d'imagerie ;
un module de calcul ;
un module de visualisation ; et
une mémoire comprenant des instructions qui, lorsqu'elles sont exécutées par le module informatique, amènent le module informatique à :
amener le module de visualisation à afficher un viseur circulaire pour aider un utilisateur à capturer une image de tympan à l'aide du module d'imagerie ;
déterminer une région de tympan dans l'image de tympan et calculer un pourcentage de zone de chevauchement du viseur circulaire avec la région de tympan ;
si le pourcentage est supérieur à une première valeur prédéterminée, amener le module de visualisation à afficher un premier signe pour informer l'utilisateur ;
et,
si le pourcentage est inférieur à une deuxième valeur prédéterminée, amener le module de visualisation à afficher un deuxième signe pour guider l'utilisateur pour déplacer l'otoscope dans un sens spécifique.

9. Otoscope selon la revendication 8, le viseur circulaire affiché sur le module de visualisation comprenant un viseur interne pour viser les osselets auditifs.

10. Otoscope selon la revendication 8, la région du tympan étant déterminée par un procédé comprenant les étapes de calcul de valeurs de contraste de l'image du tympan et de définition d'une limite entre un tympan et un conduit auditif dans l'image du tympan.

11. Otoscope selon la revendication 8, la première valeur prédéterminée étant de 20%, de 25% ou de 30%.

12. Otoscope selon la revendication 8, le premier signe comprenant un changement de couleur du viseur circulaire.

13. Otoscope selon la revendication 8, la deuxième valeur prédéterminée étant d'au moins 70%, 80%, 90%, ou 95% et le deuxième signe comprenant une flèche indiquant le sens spécifique.
